Europäisches Patentamt

European Patent Office

Office européen des brevets

(11) Veröffentlichungsnummer: **0 056 491**
**B1**

(19)

(12) # EUROPÄISCHE PATENTSCHRIFT

(45) Veröffentlichungstag der Patentschrift:
**21.12.83**

(21) Anmeldenummer: **81110788.7**

(22) Anmeldetag: **25.12.81**

(51) Int. Cl.³: **C 07 C 87/60**, C 07 C 85/04

(54) Verfahren zur Herstellung von grobkörnigem Triaminotrinitrobenzol.

(30) Priorität: **21.01.81 DE 3101783**

(43) Veröffentlichungstag der Anmeldung:
**28.07.82 Patentblatt 82/30**

(45) Bekanntmachung des Hinweises auf die Patenterteilung:
**21.12.83 Patentblatt 83/51**

(84) Benannte Vertragsstaaten:
**CH FR GB LI SE**

(56) Entgegenhaltungen:
**US - A - 3 394 183**
**US - A - 4 032 377**

**Chemical Abstracts Band 94, Nr. 19 11. Mai 1981, Columbus, Ohio, USA, J.G. LOCKE "Low chlorine sym-triamino-trinitrobenzene (TATB) produced by emulsion amination" Seite 615, Spalte 1, Abstract Nr. 156440v**

(73) Patentinhaber: **Fraunhofer-Gesellschaft zur Förderung der angewandten Forschung e.V., Leonrodstrasse 54, D-8000 München 19 (DE)**

(72) Erfinder: **Hansen, Rolf, Dr. Dipl.-Chem., Grenzweg 4, D-7507 Pfinztal 1 (DE)**
Erfinder: **Engel, Walter, Dr. Dipl.-Chem., Frühlingstrasse 21, D-7507 Pfinztal 1 (DE)**
Erfinder: **Schubert, Hiltmar, Dr. rer. nat., Dahlienweg, D-7519 Walzbachtal (DE)**

(74) Vertreter: **Dr.-Ing. Hans Lichti Dipl.-Ing. Heiner Lichti Dipl.-Phys. Jost Lempert, Postfach 41 07 60 Durlacher Strasse 31, D-7500 Karlsruhe 41 (DE)**

Verfahren zur Herstellung von grobkörnigem Triaminotrinitrobenzol

Die Erfindung betrifft ein Verfahren zur Herstellung von grobkörnigem 1,3,5-Triaminotrinitrobenzol (TATB) durch Aminierung, z. B. mittels $NH_3$, von 1,3,5-Trichlortrinitrobenzol (TCTNB) bei erhöhter Temperatur.

Auf dem Gebiet der Explosivstoffe hat in neuerer Zeit Triaminotrinitrobenzol als Spezialsprengstoff erhöhte Bedeutung gewonnen. Die Ursache hierfür ist die außerordentliche Unempfindlichkeit gegenüber Schockbelastungen sowie die höhere Dichte und größere Detonationsgeschwindigkeit gegenüber dem »klassischen« Sprengstoff TNT. Einer breiten Anwendung dieses Spezialsprengstoffes standen bisher jedoch die enorm hohen Herstellungskosten im Weg, die ein Vielfaches herkömmlicher Sprengstoffe betragen. Bei der Herstellung von TATB geht man im allgemeinen von Trichlortrinitrobenzol (TCTNB) aus, da dieser Ausgangsstoff — im Gegensatz beispielsweise zu Tribromtrinitrobenzol — relativ billig ist. Nach einem älteren Verfahren (Rev. trav. chim. 56 [1937], 1175) wird die Aminierung von TCTNB durch Reaktion von wäßriger Ammoniaklösung in niedrigsiedenden Lösungsmitteln, wie Ethanol oder Toluol, durchgeführt. Nach einer relativ langen Reaktionszeit entsteht ein sehr feines Produkt mit einer mittleren Korngröße von 10 µm. Derart feinkörniges Produkt läßt sich nur schwer verarbeiten.

Soll sich der Sprengstoff durch Gießen oder Pressen verarbeiten lassen, wie dies heute meist gefordert wird, so ist ein grobkörniges Produkt mit einer mehrmodalen Kornverteilung zwischen 40 und 250 µm wünschenswert, um einerseits entsprechende Viskositäten in der Gießmischung zu erhalten, andererseits den Anteil eventueller Zusatzstoffe, die für das Gießen oder Verpressen notwendig sind, möglichst gering zu halten und damit eine hohe Sprengstoffdichte zu erreichen.

Um diesen Zielen näherzukommen, ist ein Verfahren bekanntgeworden (US-PS 4 032 377), bei dem das zuvor beschriebene Verfahren auf einem höheren Temperaturniveau bis 150°C geführt wird. Voraussetzung hierfür ist allerdings, daß auch der Druck an diese Temperatur angepaßt wird, so daß also das Verfahren unter nennenswertem Überdruck abläuft. Mit diesem Verfahren wird ein grobkörnigeres Material erhalten, das somit günstigere Verarbeitungseigenschaften aufweist. Dieses an sich positive Ergebnis wird allerdings durch die Anwendung des hohen Drucks mit entsprechend größerem apparativem Aufwand erkauft. Dadurch steigen die ohnehin schon hohen Herstellungskosten weiter an.

Aufbauend auf diesem Verfahren liegt der Erfindung die Aufgabe zugrunde, für die Reaktion Verfahrensparameter aufzuzeigen, die es ermöglichen, bei normalem apparativen Aufwand und kurzen Reaktionszeiten grobkörniges TATB zu erhalten.

Ausgehend von dem eingangs angedeuteten Verfahren wird diese Aufgabe dadurch gelöst, daß Aminierungsmittel verwendet werden, deren Zersetzungstemperatur oberhalb 160°C liegt und daß die Reaktion oberhalb dieser Temperatur bei Normaldruck unter Anwesenheit wenigstens eines Antioxidans geführt wird.

Praktische Versuche haben gezeigt, daß bei einer Reaktionsführung oberhalb 160°C, also einer Temperatur, die im Bereich der Bildungstemperatur von TATB liegt, ein ausreichend grobkörniges Produkt auch dann erhalten werden kann, wenn drucklos gearbeitet wird. Um die bei dieser hohen Temperatur drohende Zersetzung der Substanz zu verhindern, die sich in einer zunehmend dunklen Verfärbung des Produktes zeigt, werden Antioxidantien, z. B. Ethyltriphenylharnstoff, in geringen Mengen zugegeben. Durch Einhalten kurzer Reaktionszeiten und unmittelbare Einbringung der Aminierungsmittel und Antioxidantien ist es ohne weiteres möglich, die Reaktion bei Normaldruck unter Kontrolle zu halten.

Als Aminierungsmittel, welche die vorgenannte Bedingung hinsichtlich der Zersetzungstemperatur erfüllen, kommen Ammoniumacetat, Ammoniumoxalat, Ammoniumcarbamat, Harnstoff oder aber auch Ammoniak in Frage.

Die Reaktionsführung bei Temperaturen oberhalb 160°C läßt sich verfahrenstechnisch auf verschiedene Weise verwirklichen:

Beispielsweise kann TCTNB geschmolzen und der Schmelze das Aminierungsmittel in festem, geschmolzenem oder gasförmigem Zustand zugegeben werden. Statt dessen kann auch das Aminierungsmittel auf eine Temperatur nahe seiner Zersetzungstemperatur erhitzt und TCTNB in fester oder flüssiger Form zugegeben werden.

In bevorzugter Ausführung jedoch ist vorgesehen, daß TCTNB in einem Lösungsmittel, dessen Siedetemperatur höher als 160°C liegt, gelöst und der Lösung bei einer Temperatur oberhalb 160°C das Aminierungsmittel zugegeben wird.

Bei dieser Verfahrensführung fallen das sich bildende TATB sowie $NH_4Cl$ aus, während TCTNB in Lösung bleibt. Das Lösungsmittel wird so ausgewählt, daß es sich gegenüber TCTNB inert verhält. Hierbei ergeben sich stark verkürzte Reaktionszeiten. Ferner wird ein Ansteigen der mittleren Korngröße auf Kosten kleiner Korngrößen beobachtet, wenn das TATB in dem Reaktionsmedium etwas löslich ist. Überraschend ist hierbei, daß die Art des Lösungsmittels die Bildungstemperatur von TATB beeinflußt, so daß umgekehrt die Entstehung von Zersetzungsprodukten durch Auswahl des Lösungsmittels gesteuert werden kann.

Als besonders günstige Lösungsmittel haben sich Trioctylphosphat, Siliconöl, Dibutylphthalat, Decalin, Nitrobenzol oder Paraffinöl erwiesen.

Nachstehend sind einige Ausführungsbeispiele des erfindungsgemäßen Verfahrens in ihren Parametern und mikrophotographischen Abbildungen des Korns wiedergegeben:

## Beispiel 1

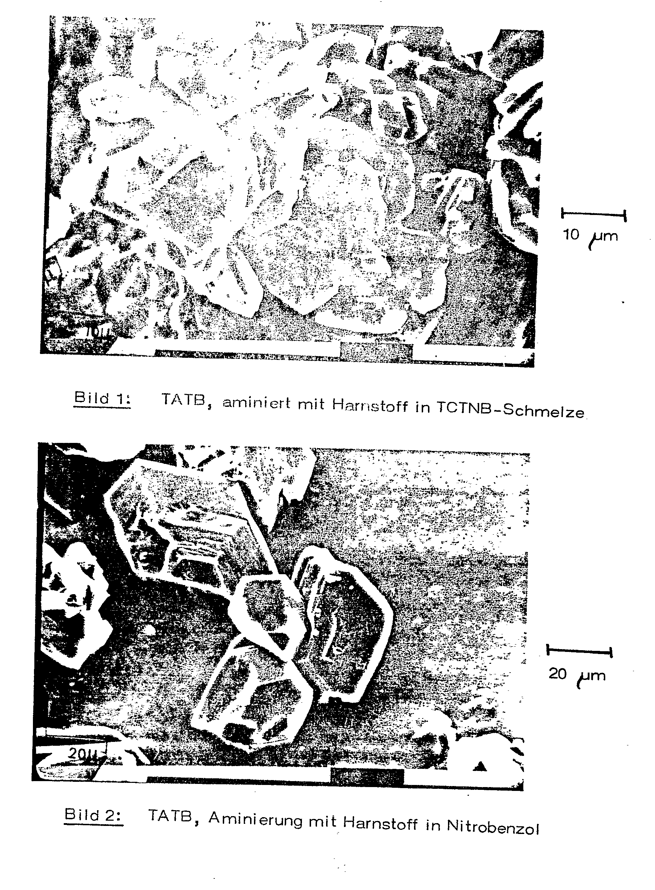

In einem heizbaren Gefäß werden 0,1 kg TCTNB mit 2 g Ethyltriphenylharnstoff bei 190°C geschmolzen und schnell mit 0,15 kg geschmolzenem Harnstoff versetzt. Nach einer Reaktionszeit von 3 Minuten wird die Mischung schnell abgekühlt und mit Wasser—Aceton gereinigt. Man erhält folgendes Ergebnis (Bild 1):

| | |
|---|---|
| Ausbeute | 75% d. Th. |
| Dichte | 1,93 g/cm³ |
| Korngröße | 50 µm |
| Chloridgehalt | >0,01% |
| Zersetzungspunkt | 361°C |

(n. DTA = Differentialthermoanalyse)

## Beispiel 2

Die Aminierung von TCTNB wurde bei einer Reaktionstemperatur oberhalb von 160°C in Nitrobenzol (oder siedendem Chlorbenzol) durchgeführt. In 1 kg heißem Nitrobenzol werden 0,5 kg TCTNB mit 10 g Ethyltriphenylharnstoff und 0,75 kg Harnstoff gelöst. Oberhalb 150°C beginnt TATB auszufallen. Entsprechend der eingestellten Reaktionstemperatur ist der Prozeß in 2 Stunden beendet. Der Niederschlag wird mit Aceton und Wasser gewaschen, bis keine chloridhaltigen Verbindungen nachweisbar sind. Folgendes Ergebnis wurde erhalten (Bild 2):

| | |
|---|---|
| Ausbeute | 65% |
| Chloridgehalt | >0,01% |
| Dichte | 1,84 g/cm³ |
| Korngröße | 150 µm |
| Zersetzungspunkt | 359°C |

(n. DTA)

## Beispiel 3

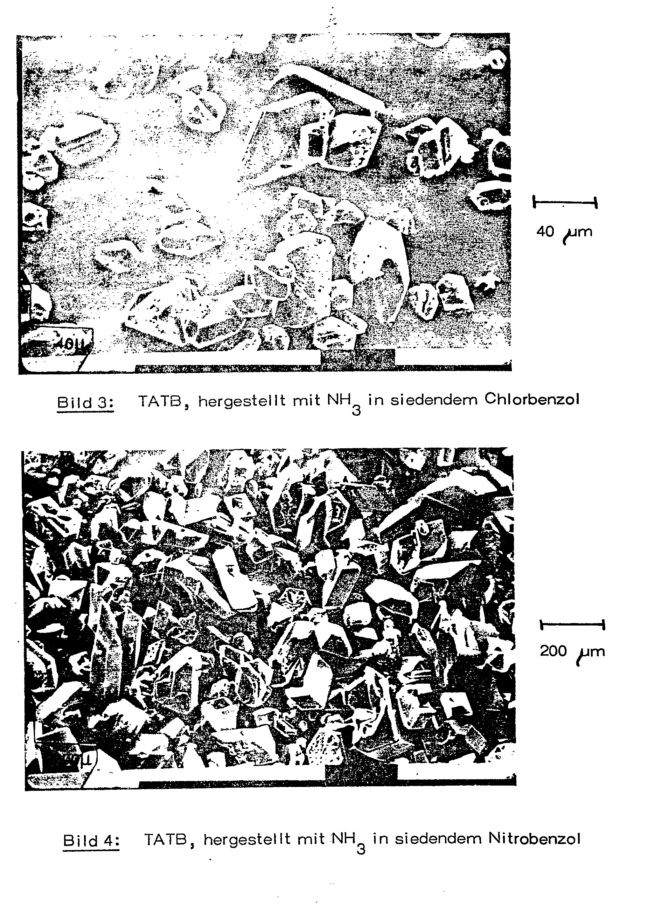

Die Aminierung von 0,5 kg TCTNB wird in 1 kg heißem Nitrobenzol oder siedendem Chlorbenzol durch Einleiten von gasförmigem Ammoniak unter Zusatz von 10 g Ethyltriphenylharnstoff durchgeführt. Unter Berücksichtigung der gewählten Temperatur fällt das TATB innerhalb von 2 Stunden aus. Der Niederschlag wird mit Aceton und Wasser gewaschen, bis keine chloridhaltigen Verbindungen nachweisbar sind. Folgendes Ergebnis wurde erhalten (Bild 3, 4):

| | |
|---|---|
| Ausbeute | 55% |
| Chloridgehalt | >0,01% |
| Dichte | 1,90 g/cm³ |
| Korngröße | 50—300 µm |
| Zersetzungspunkt | 358°C |

## Patentansprüche

1. Verfahren zur Herstellung von grobkörnigem 1,3,5-Triaminotrinitrobenzol (TATB) durch Aminierung, z. B. mittels $NH_3$, von 1,3,5-Trichlortrinitrobenzol (TCTNB) bei erhöhter Temperatur, dadurch gekennzeichnet, daß Aminierungsmittel verwendet werden, deren Zersetzungstemperatur oberhalb 160°C liegt und daß die Reaktion oberhalb dieser Temperatur bei Normaldruck unter Anwesenheit wenigstens eines Antioxidans geführt wird.

2. Verfahren nach Anspruch 1, dadurch gekennzeichnet, daß als Aminierungsmittel Ammoniumacetat, Ammoniumoxalat, Ammoniumcarbamat, Harnstoff oder Ammoniak verwendet werden.

3. Verfahren nach Anspruch 1 oder 2, dadurch gekennzeichnet, daß TCTNB geschmolzen und der Schmelze das Aminierungsmittel in festem, geschmolzenem oder gasförmigem Zustand zugegeben werden.

4. Verfahren nach Anspruch 1 oder 2, dadurch gekennzeichnet, daß das Aminierungsmittel auf eine Temperatur nahe seiner Zersetzungstemperatur erhitzt und TCTNB in fester oder flüssiger Form zugegeben wird.

5. Verfahren nach Anspruch 1 oder 2, dadurch gekennzeichnet, daß TCTNB in einem Lösungsmittel, dessen Siedetemperatur höher als 160°C liegt, gelöst und der Lösung bei einer Temperatur oberhalb 160°C das Aminierungsmittel zugegeben wird.

6. Verfahren nach Anspruch 5, dadurch gekennzeichnet, daß als Lösungsmittel Trioctylphosphat, Siliconöl, Dibutylphthalat, Decalin, Nitrobenzol oder Paraffinöl verwendet werden.

7. Verfahren nach einem der Ansprüche 1 bis 6, dadurch gekennzeichnet, daß als Antioxidans Ethyltriphenylharnstoff zugegeben wird.

## Claims

1. A process for the manufacture of coarse 1,3,5-triaminotrinitrobenzene (TATB) by aminating 1,3,5-trichlortrinitrobenzene (TCTNB), for example with $NH_3$, at elevated temperature, wherein aminating agents having a decomposition temperature of above 160°C are used and wherein aminating is effected at a temperature greater than 160°C and at atmospheric pressure in the presence of at least one antioxidant.

2. The process according to claim 1 wherein ammonium acetate, ammonium oxalate, ammonium carbamate, urea or ammonia is used as aminating agent.

3. The process according to claim 1 or 2 wherein TCTNB is a melt and wherein said aminating agent is a solid, melt or gas added to said melt.

4. The process according to claim 1 or 2 wherein said aminating agent is heated to a temperature near its decomposition temperature and wherein TCTNB is added in a solid or liquid state.

5. The process according to claim 1 or 2 comprising dissolving TCTNB in a solvent having a boiling point greater than 160°C and adding to this solution said aminating agent at a temperature greater than 160°C.

6. The process according to claim 5 wherein said solvent is trioctylphosphate, silicon oil, dibutylphthalate, Decalin, nitrobenzene or paraffin oil.

7. The process according to one of the claims 1 to 6 wherein as an antioxidant ethyltriphenylurea is added.

## Revendications

1. Procédé de préparation de 1,3,5-triaminotrinitrobenzène (TATB) à gros grains par amination, par exemple au moyen de $NH_3$, de 1,3,5-trichlorotrinitrobenzène (TCTNB) à température élevée, caractérisé en ce qu'on utilise des agents d'amination dont la température de décomposition se situe au-dessus de 160°C, et qu'on conduit la réaction au-dessus de cette température à pression normale en présence d'au moins un anti-oxydant.

2. Procédé selon la revendication 1, caractérisé en ce qu'on utilise comme agent d'amination l'acétate d'ammonium, l'oxalate d'ammonium, le carbamate d'ammonium, l'urée ou l'ammoniac.

3. Procédé selon la revendication 1, caractérisé en ce qu'on fait fondre le TCTNB et qu'on y ajoute l'agent d'amination à l'état solide, fondu ou gazeux.

4. Procédé selon la revendication 1 ou 2, caractérisé en ce qu'on chauffe l'agent d'amination à une température voisine de sa température de décomposition et qu'on ajoute le TCTNB sous forme solide ou liquide.

5. Procédé selon la revendication 1 ou 2, caractérisé en ce qu'on dissout le TCTNB dans un solvant dont la température d'ébullition est supérieure à 160°C et qu'on ajoute l'agent d'amination à la solution à une température supérieure à 160°C.

6. Procédé selon la revendication 5, caractérisé en ce qu'on utilise comme solvant le phosphate de trioctyle, l'huile de silicone, le phthalate de dibutyle, la décaline, le nitrobenzène ou l'huile de paraffine.

7. Procédé selon l'une des revendications 1 à 6, caractérisé en ce qu'on ajoute comme anti-oxydant de l'éthyltriphénylurée.

Bild 1: TATB, aminiert mit Harnstoff in TCTNB-Schmelze

Bild 2: TATB, Aminierung mit Harnstoff in Nitrobenzol

5

Bild 3: TATB, hergestellt mit $NH_3$ in siedendem Chlorbenzol

Bild 4: TATB, hergestellt mit $NH_3$ in siedendem Nitrobenzol